# EUROPEAN PATENT APPLICATION

(11) **EP 1 388 544 A1**
(43) Date of publication of application: **11.02.2004**
(21) Application number: 02447150.0
(22) Date of filing: 31.07.2002
(51) Int. Cl.: C07K 16/42, C07K 14/705, C12N 5/16, A61K 38/17, A61K 38/08

(54) **Anti-idiotypic antibodies against factor VIII inhibitor and uses thereof**

(71) Applicant: D. Collen Research Foundation vzw, 3000 Leuven (BE)
(72) Inventor: Gille, Jean Guy G., 1090 Bruxelles (BE); Saint-Remy, Jean-Marie R., 1390 Grez-Doiceau (BE); Jacquemin, Marc G., 5330 Sart-Bernard (BE)
(74) Representative: Hertoghe, Kris Angèle Louisa

(57) **Abstract**

The present invention discloses anti-idiotypic antibodies and fragments thereof against inhibitory Factor VIII antibodies, said inhibitory antibodies having an affinity for the C2 domain of Factor VIII. The anti-idiotypic antibodies of the present invention are able to completely neutralize in vitro and in an in vivo mouse model the inhibitory activity of FVIII inhibitors. The anti idiotypic antibodies of the present invention can be applied for the prevention, treatment or reduction of bleeding disorders of haemophilia patients with inhibitory antibody against the C2 domain of Factor VIII.

SEQUENCE LISTING

<110> D. Collen Research Foundation vzw
Gilles, Jean Guy G
Jacquemin, Marc
Saint-Remy, Jean-Marie R

<120> Anti-idiotypic antibodies against Factor VIII inhibitor and
uses thereof

<130> C 2104 EP

<160> 10

<170> PatentIn version 3.1

<210> 1
<211> 411
<212> DNA
<213> Mus musculus

<220>
<221> CDS
<222> (1)..(411)
<223>

<220>
<221> misc_feature
<222> (78)..(111)
<223> CDR1

<220>
<221> misc_feature
<222> (150)..(198)
<223> CDR2

<220>
<221> misc_feature
<222> (297)..(333)
<223> CDR2

<220>
<221> V_region
<222> (1)..(411)
<223> 14C12 monoclonal antibody heavy chain

<400> 1
gag gtc cag ctt cag cag tct gga cct gag ctg gtt aag cct ggg gct 48
Glu Val Gln Leu Gln Gln Ser Gly Pro Glu Leu Val Lys Pro Gly Ala
1               5                   10                  15

tca gtg aag ctg tcc tgc aag gct tct gga tac aca ttc act agc tct 96
Ser Val Lys Leu Ser Cys Lys Ala Ser Gly Tyr Thr Phe Thr Ser Ser
            20                  25                  30

gtt atg cac tgg ctg aag cag aag tct ggg cag ggc ctt gag tgg att 144
Val Met His Trp Leu Lys Gln Lys Ser Gly Gln Gly Leu Glu Trp Ile
        35                  40                  45

gga tat att aat cct tac aat gat ggt act aag tac aat gag aag ttc 192
Gly Tyr Ile Asn Pro Tyr Asn Asp Gly Thr Lys Tyr Asn Glu Lys Phe

50                    55                    60

aca gcc aag gcc aca ctg act tca gac aaa tcc tcc agc aca gtc tac          240
Thr Ala Lys Ala Thr Leu Thr Ser Asp Lys Ser Ser Ser Thr Val Tyr
65                  70                  75                  80

atg gag ctc agc ggc ctg acc tct gag gac ttt gcg gtc tat tac tgt          288
Met Glu Leu Ser Gly Leu Thr Ser Glu Asp Phe Ala Val Tyr Tyr Cys
                85                  90                  95

gca cga tcg gga ggt tta cta cga ggt tac tgg tac ttc gat gtc tgg          336
Ala Arg Ser Gly Gly Leu Leu Arg Gly Tyr Trp Tyr Phe Asp Val Trp
            100                 105                 110

ggc gca ggg acc acg gtc acc gtc tcc tca gcc aaa aca aca gcc cca          384
Gly Ala Gly Thr Thr Val Thr Val Ser Ser Ala Lys Thr Thr Ala Pro
        115                 120                 125

tcg gtc tat ccc ttg gtc cct ggc tgc                                      411
Ser Val Tyr Pro Leu Val Pro Gly Cys
    130                 135

<210>  2
<211>  137
<212>  PRT
<213>  Mus musculus
<220>
<221>  misc_feature
<222>  (78)..(111)
<223>  CDR1

<220>
<221>  misc_feature
<222>  (150)..(198)
<223>  CDR2

<220>
<221>  misc_feature
<222>  (297)..(333)
<223>  CDR2

<400>  2

Glu Val Gln Leu Gln Gln Ser Gly Pro Glu Leu Val Lys Pro Gly Ala
1               5                   10                  15

Ser Val Lys Leu Ser Cys Lys Ala Ser Gly Tyr Thr Phe Thr Ser Ser
            20                  25                  30

Val Met His Trp Leu Lys Gln Lys Ser Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Thr Ala Lys Ala Thr Leu Thr Ser Asp Lys Ser Ser Ser Thr Val Tyr

65                  70                  75                  80

Met Glu Leu Ser Gly Leu Thr Ser Glu Asp Phe Ala Val Tyr Tyr Cys
                85                  90                  95

Ala Arg Ser Gly Gly Leu Leu Arg Gly Tyr Trp Tyr Phe Asp Val Trp
            100                 105                 110

Gly Ala Gly Thr Thr Val Thr Val Ser Ser Ala Lys Thr Thr Ala Pro
        115                 120                 125

Ser Val Tyr Pro Leu Val Pro Gly Cys
    130                 135

<210>  3
<211>  369
<212>  DNA
<213>  Mus musculus

<220>
<221>  CDS
<222>  (1)..(369)<223>

<220>
<221>  misc_feature
<222>  (72)..(102)
<223>  CDR1

<220>
<221>  misc_feature
<222>  (150)..(168)
<223>  CDR2

<220>
<221>  misc_feature
<222>  (267)..(291)
<223>  CDR3

<220>
<221>  V_region
<222>  (1)..(369)
<223>  14C12 monoclonal antibody light chain

<400>  3
gat ctt gtg cta act cag tct cca gcc acc ctg tct gtg act cca gga        48
Asp Leu Val Leu Thr Gln Ser Pro Ala Thr Leu Ser Val Thr Pro Gly
1               5                   10                  15

gat agt gtc agt ctt tcc tgt agg gcc agc caa gat att acc aac acc        96
Asp Ser Val Ser Leu Ser Cys Arg Ala Ser Gln Asp Ile Thr Asn Thr
            20                  25                  30

ctt cac tgg tat cat caa aaa tca cat gag tct cca agg ctt ctc atc       144
Leu His Trp Tyr His Gln Lys Ser His Glu Ser Pro Arg Leu Leu Ile
        35                  40                  45

aag tat gtt tcc cag tcc atc tct ggg atc ccc tcc agg ttc agt ggc     192
Lys Tyr Val Ser Gln Ser Ile Ser Gly Ile Pro Ser Arg Phe Ser Gly
    50                  55                  60

agt gga tca ggg aca gtt ttc act ctc agt atc aac agt gtg gag act     240
Ser Gly Ser Gly Thr Val Phe Thr Leu Ser Ile Asn Ser Val Glu Thr
65                  70                  75                  80

gaa gat ttt gga gtg tat ttc tgt cag cag agt acc agc tgg ccg tac     288
Glu Asp Phe Gly Val Tyr Phe Cys Gln Gln Ser Thr Ser Trp Pro Tyr
                85                  90                  95

aca ttc gga ggg ggg acc aag ttg gaa ata aaa cgg gct gat gct gca     336
Thr Phe Gly Gly Gly Thr Lys Leu Glu Ile Lys Arg Ala Asp Ala Ala
            100                 105                 110

cca act gta tcc atc ttc cca cca tcc agt gag                         369
Pro Thr Val Ser Ile Phe Pro Pro Ser Ser Glu
        115                 120

<210>  4
<211>  123
<212>  PRT
<213>  Mus musculus

<220>
<221>  misc_feature
<222>  (72)..(102)
<223>  CDR1

<220>
<221>  misc_feature
<222>  (150)..(168)
<223>  CDR2

<220>
<221>  misc_feature
<222>  (267)..(291)
<223>  CDR3

<400>  4

Asp Leu Val Leu Thr Gln Ser Pro Ala Thr Leu Ser Val Thr Pro Gly
1               5                   10                  15

Asp Ser Val Ser Leu Ser Cys Arg Ala Ser Gln Asp Ile Thr Asn Thr
            20                  25                  30

Leu His Trp Tyr His Gln Lys Ser His Glu Ser Pro Arg Leu Leu Ile
        35                  40                  45

Lys Tyr Val Ser Gln Ser Ile Ser Gly Ile Pro Ser Arg Phe Ser Gly
    50                  55                  60

Ser Gly Ser Gly Thr Val Phe Thr Leu Ser Ile Asn Ser Val Glu Thr
65                  70                  75                  80

Glu Asp Phe Gly Val Tyr Phe Cys Gln Gln Ser Thr Ser Trp Pro Tyr
                85                  90                  95

Thr Phe Gly Gly Gly Thr Lys Leu Glu Ile Lys Arg Ala Asp Ala Ala
            100                 105                 110

Pro Thr Val Ser Ile Phe Pro Pro Ser Ser Glu
        115                 120

<210>  5
<211>  12
<212>  PRT
<213>  Mus musculus
<400>  5

Gly Tyr Thr Phe Thr Ser Ser Val Met His Trp Leu
1               5                   10

<210>  6
<211>  18
<212>  PRT
<213>  Mus musculus
<400>  6

Gly Tyr Ile Asn Pro Tyr Asn Asp Gly Thr Lys Tyr Asn Glu Lys Phe
1               5                   10                  15

Thr Ala

<210>  7
<211>  13
<212>  PRT
<213>  Mus musculus
<400>  7

Ser Gly Gly Leu Leu Arg Gly Tyr Trp Tyr Phe Asp Val
1               5                   10

<210>  8
<211>  11
<212>  PRT
<213>  Mus musculus
<400>  8

Arg Ala Ser Gln Asp Ile Thr Asn Thr Leu His
1               5                   10

<210>  9
<211>  7
<212>  PRT

<213>  Mus musculus
<400>  9

Tyr Val Ser Gln Ser Ile Ser
1               5


<210>  10
<211>  9
<212>  PRT
<213>  Mus musculus
<400>  10

Gln Gln Ser Thr Ser Trp Pro Tyr Thr
1               5

## Description

### FIELD OF THE INVENTION

The present invention relates to pharmaceutical compositions for the treatment of haemophilia, especially for the treatment of human patients who have developed FVIII inhibitors directed to the C2 domain. The present invention provides anti-idiotypic antibodies against FVIII inhibitors directed to the C2 domain. The invention further relates to monoclonal cell lines expression such anti-idiotypic antibodies. The invention also relates to pharmaceutical compositions comprising the anti-idiotypic antibodies of the present invention.

### BACKGROUND OF THE INVENTION

Haemophilia A is an X-linked disorder characterised by the absence or insufficient amount of functional factor VIII, a 330kD glycoprotein molecule produced by the liver as a single polypeptide chain of 2332 amino acids. This deficiency affects 1 in 10,000 males and can result in uncontrolled bleeding in joints, muscles and soft tissues. Patients affected by the severe form of the disease (FVIII activity lower than 1% of normal level) suffer from spontaneous bleedings. Patients with corresponding FVIII activity from 1 to 5%, or higher than 5% are defined as moderate or mild haemophilia A, respectively, and suffer from limited bleeding occurring after minor trauma or surgery. The coagulation pathway is restored by administration of FVIII concentrates prepared from plasma or produced by recombinant cDNA technology.

The human FVIII gene has been isolated and expressed in mammalian cells, as reported by various authors, including Wood et al. in *Nature* (1984) **312**:330-337 and the amino-acid sequence was deduced from cDNA. U.S. Patent No. 4,965,199 discloses a recombinant DNA method for producing FVIII in mammalian host cells and purification of human FVIII. The human FVIII detailed structure has been extensively investigated. The cDNA nucleotide sequence encoding human FVIII and predicted amino-acid sequence have been disclosed for instance in U.S. Patent No. 5,663,060. In a FVIII molecule, a domain may be defined as a continuous sequence of amino-acids that is defined by internal amino-acid sequence homology and sites of proteolytic cleavage by a suitable protease such as thrombin. Unless otherwise specified, FVIII domains include the following amino-acid residues, when the sequences are aligned with the human amino-acid sequence: A1, residues 1-372; A2, residues 373-740; B, residues 741-1648; A3, residues 1690-2019; C1, residues 2020-2172; C2, residues 2173-2332. The remaining sequence, residues 1649-1689, is usually referred to as the FVIII light chain activation peptide. FVIII is produced as a single polypeptide chain which, upon processing within the cell, is rapidly cleaved after secretion to form a heterodimer made of a heavy chain containing the A1, A2 and B domains and a light chain made of the A3-C1-C2 domains, according to Kaufman et al., *J. Biol. Chem.* (1988) **263**:6352-6362. The two chains are non-covalently bound by divalent cations. Both the single-chain polypeptide and the heterodimer circulate in plasma as inactive precursors, as taught by Ganz et al., *Eur. J. Biochem.* (1988) **170**:521-528. Activation of factor VIII in plasma initiates by thrombin cleavage between the A2 and B domains, which releases the B domain and results in a heavy chain consisting of the A1 and A2 domains, according to Eaton et al., *Biochemistry* (1986) **25**:505-512. Human recombinant FVIII may be produced by genetic recombination in mammalian cells such as CHO (Chinese Hamster Ovary) cells, BHK (Baby Hamster Kidney) cells or other equivalent cells.

Pratt et al. in *Nature* (1999) **402**:439-42 disclose the detailed structure of the carboxy-terminal C2 domain of human FVIII, which contains sites that are essential for its binding to von Willebrand factor and to negatively charged phospholipid surfaces. This structure, which reveals a beta-sandwich core from which two beta-turns and a loop display a group of solvent-exposed hydrophobic residues, partly explains mutations in the C2 region that lead to bleeding disorders in haemophilia A. According to Gale et al. in *Thromb. Haemost.* (2000) 83:78-85, of the at least 250 missense mutations that cause FVIII deficiency and haemophilia A, 34 are in the C domains.

FVIII is a cofactor of the intrinsic pathway of the coagulation cascade, which acts by increasing the proteolytic activity of activated factor IX over factor X, in the so-called tenase complex formation. Patients suffering from haemophilia A present with bleedings which are either spontaneous in the severe form of the disease, or occur after trauma in the mild/moderate forms.

Haemophilia A patients are usually treated by replacement therapy, which consists in infusing human FVIII either purified from pools of donor plasma, or obtained by cDNA recombination technology.

The majority of the patients are immunologically unresponsive to these infusions, but for yet unclear reasons, 25% of them mount an IgG immune response towards FVIII, which can result in complete inhibition of the procoagulant activity of infused FVIII (Briët E et al. in (1994) *Throm. Haemost.;* **72**: 162-164 ; Ehrenforth S et al. in (1992) *Lancet;* **339**:594). Such specific IgG, which belong to the IgG 1, 2, 4 subclasses, are called FVIII inhibitors. Published studies have demonstrated that the anti-FVIII immune response is polyclonal, and primarily directed towards the A2, A3 and C2 domains (Scandella D et al. in (1989) *Blood;* **74:** 1618-1626; Gilles JG et al. in (1993) *Blood;* **82:** 2452-2461).

Recent studies using human monoclonal antibodies derived from the peripheral memory B cell repertoire of inhibitor patients indicated that important epitopes are also located on the C1 domain (Jacquemin M et al. in (2000) *Blood* **95**:156-163). The mechanism by which anti-FVIII antibodies interfere with the function of FVIII are numerous, including proteolytic cleavage of FVIII and interaction with different partners such as von Willebrand factor (VWF), phospholipids, FIX, FXa or APC. Most of such mechanisms are now well described in studies using mouse or human anti-FVIII antibodies. Thus, antibodies can reduce the rate at which FVIII is activated by either binding to a proteolytic cleavage site or by inducing a 3D conformational change in FVIII that renders it less amenable to proteolysis. Antibodies interfering with the binding of VWF to FVIII appear to be very efficient as inhibitors, as shown in recent studies using human monoclonal antibodies directed towards the C2 domain, which is one of the major VWF binding sites (Jacquemin M et al in (1998) *Blood;* 92:496-501). Suppressing the production of inhibitors and establishing a state of immune unresponsiveness to FVIII remains a major goal. The medical community is, however, far from matching such goals, due basically to the limited understanding of the mechanisms underlying specific antibody production and regulation.

Presently, to control such an immune response, several treatments are used including bypassing agents such as desmopressin (DDAVP), agents promoting coagulation such as prothrombin complex concentrates (PCC) or activated PCC, recombinant FVIIa, plasmapheresis, infusions of large or intermediate doses of FVIII (200-300 IU/kg body weight or 25-50 IU/kg body weight, respectively). However, none of these methods are satisfactory and in all cases are of extremely high cost.

Based on these observations and on the understanding of the mechanisms of immune tolerance, anti-idiotypic antibodies appear to be a promising way of treating inhibitors. In fact, it is well established that tolerance to self-protein is first induced at an early stage by clonal deletion of self-reactive B and T cells in the bone marrow and the thymus, respectively. However, not all self-reactive lymphocytes are eliminated by central deletion. Auto-reactive B cells are a common feature of peripheral blood, as well as low- or intermediate-affinity self-reactive T cells. A number of mechanisms by which such auto-reactive cells are rendered non-functioning or are deleted in the periphery have been described. Anti-idiotypic antibodies can represent a third level of tolerance maintenance in this general scheme, with their capacity to fine tuning the function of antibodies and maintain a subtle equilibrium between complementary idiotypes expressed on B and T cells.

A good indication of how anti-idiotypic antibodies can exert a regulatory mechanism in the periphery is provided by the demonstration that healthy individuals with normal levels of FVIII produce significant titres of inhibitory antibodies to FVIII (Algiman M et al. in (1994) *Proc Natl Acad Sci USA.;* **89** :3795-3799; Gilles JG et al in (1994) J *Clin Invest.* **94**:1496-505), the activity of which is undetectable in plasma because of the presence of complementary anti-idiotypic antibodies. However, such a FVIII inhibitory activity can be readily detected when anti-FVIII antibodies are purified by a combination of chromatography and specific immunoadsorption over insolubilized FVIII. The FVIII inhibitory capacity of affinity-purified antibodies was demonstrated to be equal to that of anti-FVIII antibodies purified from haemophilia A patient's plasma with high level of inhibitors, as measured by Bethesda assay (Gilles JG et al. in (1994) J *Clin Invest.;* **94**:1496-505).

Such neutralising anti-idiotypic activity has also been detected in a group of patients successfully desensitised by administration of high doses FVIII (Gilles JG et al. in (1996) *J Clin Invest.;* **97**:1382-1388). The study demonstrated that the concentration of anti-FVIII antibodies, purified by the same procedure as for healthy donors, did not change during desensitisation and that antibodies maintained their capacity to inhibit the procoagulant function of FVIII, even though the titration of inhibitor using the Bethesda assay in plasma was reduced to undetectable levels. This pointed out to the potentially important function of anti-idiotypic regulation in tolerance to FVIII molecule. Therefore, any novel therapy inducing an increased production of anti-idiotypic antibodies can be a method of choice in the treatment of inhibitors. A first approach along these lines has been reported, in which patients were treated by injections of immune complexes made of FVIII and autologous specific antibodies towards FVIII, which resulted in a significant reduction in the level of circulating FVIII inhibitors, which were neutralised by corresponding anti-idiotypic antibodies (Gilles JG, Arnout J. XXI International Congress of the World Federation of Haemophilia 1994 April ; abstract). Such approach can open the way towards new therapeutic strategies for FVIII inhibitors, at potentially low cost as compared to present treatment.

Previous findings from the inventors' laboratory have shown that anti-idiotypic antibodies exert physiological properties in the homeostasis of the anti-FVIII immune response. Thus, the peripheral blood of healthy individuals contains antibodies specific for FVIII, some of which having the property of inhibiting the procoagulant function of FVIII (Gilles JGG & Saint-Remy JMR in (1994) *J Clin Invest.* **94:** 1496-1505). In such individuals, the function of FVIII is actually not altered, as antibody-mediated FVIII inhibition is neutralised by specific anti-idiotypic antibodies. We therefore concluded that anti-idiotypic antibodies have a physiological relevance in the maintenance of normal FVIII activity.

Moreover, the inventors have shown that when haemophilia A patients with inhibitor are treated by regular infusion of high doses of FVIII, a treatment also called desensitisation or tolerance induction (see above), one of the biological consequences of such infusions is the elicitation of specific anti-idiotypic antibodies able to neutralise the inhibitor (Gilles JG et al. in (1996) *J. Clin. Invest.* **97:** 1382-1388). These findings suggest that the use of anti-idiotypic antibodies could represent a valuable approach for the control of FVIII inhibitory antibodies.

The human monoclonal antibody BO2C11 is a FVIII-specific IgG4kappa antibody derived from the natural repertoire of a patient with inhibitor (Jacquemin MG, et al. in (1998) *Blood* **92:** 496-506). Ab BO2C11 recognises the C2 domain and inhibits the binding of FVIII to both von Willebrand factor (VW) and phospholipids. This antibody is representative of a major class of human inhibitory antibodies. Its mechanism of action is commonly encountered in patients with inhibitor and C2-specific antibodies are the most frequently observed inhibitory antibodies. Besides, the exact binding site of Ab BO2C11 on the C2 domain has been deciphered through X-ray analysis of crystals made of the antibody Fab fragments and the C2 domain (Spiegel P.C. Jr. et al. (2001) *Blood* **98**: 13-19).

Previous reports have described anti-idiotypic antibodies directed towards anti-FVIII antibodies. Thus, Lubahn and Reisner in (1990) *Proc. Natl. Acad. Sci. USA* **87**: 8232-8236) partially purified human anti-FVIII antibodies by salt precipitation and chromatography from the plasma of a haemophilia A patient with inhibitor. These authors demonstrated that the purified IgG recognised the native FVIII heavy chain and its thrombin-digested 43kDa chain. The preparation (SP8.4) was injected in mice in an attempt to obtain anti-idiotypic antibodies. Several clones were obtained, with one of them, Mab20-2H, inhibiting the anti-FVIII antibody binding to the heavy chain. In a functional assay, Mab20-2H did not modify the inhibitory activity of SP8.4 IgG fraction, even when added at high concentrations. Mab20-2H detected antibodies in 3.2% of the haemophilic inhibitor plasmas tested, but never neutralised the inhibitory activity. The authors concluded that Mab20-2H recognised a non-inhibitory anti-FVIII antibody that is directed toward the 43kDa chain (A2 domain of the FVIII molecule.

Another anti-idiotypic antibody (B6A2C1) was developed and described from the inventors' laboratory (Gilles JG et al in (1999) *Blood* **94,** abstract 2048, 460a). The specificity of such anti-idiotypic antibody is directed towards an anti-FVIII C1 domain inhibitor.

### SUMMARY OF THE INVENTION

It is an object of the present invention to provide a pharmaceutical composition for use on human patients suffering from haemophilia who have developed inhibitors against FVIII.

It is a further object of the present invention to provide cell lines for generating neutralising antibodies directed against human FVIII inhibitors as well as the neutralising antibodies.

In a first aspect of the present invention monoclonal anti-idiotypic antibodies against a human Factor VIII inhibitory antibody, which inhibitory antibody is directed towards the C2 domain of Factor VIII, are provided. Anti-idiotypic antibodies of the present invention are further characterised in having the capacity to neutralise by at least 50%, preferably by at least 60%, more preferably by at least 70, even more preferably by at least 80 % and most preferably by at least 90 %, the inhibition of FVIII procoagulant activity mediated by inhibitory antibodies against the C2 domain of FVIII.

In a further aspect of the present invention the heavy chain of the Factor VIII inhibitory antibody is encoded by a VH germline segment DP-5 derived from the VH1 family. In yet another aspect of the present invention the Factor VIII inhibitory antibody is Ab BO2C11.

The present invention also relates to humanised anti-idiotypic antibodies.

The present invention also relates to monoclonal anti-idiotypic antibodies, such as the one which is obtainable from cell line 14C12. The cell line 14C12 was deposited on July 30, 2002 at the Belgian Coordinated Collections of Microorganisms (BCCM), LMBP (plasmid collection, Laboratorium voor Moleculaire Biologie, Universiteit, K.L. Ledeganckstraat 35, 9000 Gent, Belgium) with Accession Number LMBP 5878CB.

The present invention further relates to anti-idiotypic antibodies wherein the variable heavy chain of anti-idiotypic antibody is encoded by the nucleotide sequence depicted in SEQ ID NO 1 or a nucleotide sequence having at least 80 % sequence identity, preferably having at least 90 % sequence identity, more preferably having at least 95% sequence identity, even more preferably having at least 99 % sequence identity with SEQ ID NO 1, and/or wherein the variable light chain of the anti-idiotypic antibody is encoded by the nucleotide sequence depicted in SEQ ID NO 3 or a nucleotide sequence having at least 80 % sequence identity, preferably having at least 90% sequence identity, more preferably having at least 95% sequence identity, even more preferably having at least 99 % sequence identity, and most preferably having at least 95 % sequence identity with SEQ ID NO 3. In particular, the nucleotide sequences may include those which use equivalent nucleotides to those specified in the SEQ ID No 1 and SEQ ID NO: 3 as determined by the redundancy of the genetic code.

The present invention further relates to anti-idiotypic antibodies having a variable heavy chain protein sequence as depicted in SEQ ID NO 2 or a protein sequence having at least 70 % sequence identity, more preferably at least 80 % sequence identity, even more preferably having at least 90% sequence identity, still even more preferably having at least 95% sequence identity, most preferably having at least 99 % sequence identity with SEQ ID NO 2 and/or having a variable light chain protein sequence as depicted in SEQ ID NO 4 or a protein sequence having at least 70 % sequence identity, more preferably at least 80 % sequence identity, even more preferably having at least 90% sequence identity, still even more preferably having at least 95% sequence identity, most preferably having at least 99 sequence identity with SEQ ID NO 4.

The present invention also relates to anti-idiotypic antibodies wherein the complementarity determining regions (CDR) of the variable heavy and/or light chains of said antibody have at least 80 % sequence identity, more preferably have at least 90 % sequence identity, even more preferably have at least 95 % sequence identity, and most preferably have at least 99 % sequence identity to the corresponding amino acid sequences depicted in SEQ ID NO 5, SEQ ID NO 6, SEQ ID NO 7, SEQ ID NO 8, SEQ ID NO 9 and SEQ ID NO 10 or are identical to the corresponding amino acid sequences depicted in SEQ ID NO 5, SEQ ID NO 6, SEQ ID NO 7, SEQ ID NO 8, SEQ ID NO 9 and SEQ ID NO 10.

The present invention also relates to modified versions of the anti-idiotypic antibodies of the present inventions and also relates to F(Ab')2 fragments, Fab' fragments, Fab fragments and other fragments comprising at least on CDR region of the anti-idiotypic antibody. The invention also relates to modified versions of said fragments.

Another aspect of the present invention is an isolated and purified peptide having a sequence selected from SEQ ID NO:5, SEQ ID NO:6, SEQ ID NO:7, SEQ ID NO:8, SEQ ID NO:9 and SEQ ID NO:10, or being at least 80 % identical in amino acid sequence to a peptide with an amino acid sequence selected of SEQ ID NO:5, SEQ ID NO:6, SEQ ID NO:7, SEQ ID NO:8, SEQ ID NO:9 and SEQ ID NO:10. The isolated and purified peptides optionally are synthetically synthesised peptides and have a degree of purity of at least 96 %, more preferably at least 98 %, and even more preferably at least 99 % as determined according to conventional methods of measuring purity.

Yet another aspect of the present invention is a monoclonal cell line expressing an anti-idiotypic antibody such as the deposited monoclonal cell line 14C12 with Accession Number LMBP 5878CB.

Another aspect of the present invention is a pharmaceutical composition comprising anti-idiotypic antibodies against inhibitory antibodies against the C2 domain of Factor VIII and fragments, peptides of said anti-idiotypic antibody and modified versions thereof, such as disclosed in details herein above.

Another aspect of the present invention is the use of an anti-idiotypic antibody or fragments, peptides of said anti-idiotypic antibody and modified versions thereof for the use as a medicine.

Another aspect of the present invention is the use of an anti-idiotypic antibody or fragments, peptides of said anti-idiotypic antibody and modified versions thereof for the manufacture of a medicament for the prevention or treatment of bleeding in a haemophilia patient with inhibitory antibody against the C2 domain of FVIII.

Another aspect of the present invention is the use of an anti-idiotypic antibody or fragments, peptides of said anti-idiotypic antibody and modified versions thereof for the manufacture of a medicament for the induction of apoptosis of B cells carrying anti C2 inhibitory antibodies. Thus the anti-idiotypic antibodies and their fragments of the present invention are capable of both suppressing immunogenic and antigenic reactions.

The present invention also relates to a method of preventing or treating the bleeding in a haemophilia patient with inhibitory antibody against the C2 domain of FVIII comprising the step of administering to a individual an anti-idiotypic antibody or fragments, peptides of said anti-idiotypic antibody and modified versions thereof.

The method also relates to a method of inducing apoptosis of B cells carrying anti-C2 inhibitory antibodies in a haemophilia patient with inhibitory antibody against the C2 domain of FVIII or fragments, peptides of said anti-idiotypic antibody and modified versions thereof.

The present invention will now be described in more details with reference to the following figures.

### BRIEF DESCRIPTION OF THE FIGURES

**Figure 1** shows the dose dependent neutralisation of the FVIII inhibition by BO2C11, neutralised by Ab 14C12 in accordance with an embodiment of the present invention.
**Figure 2** shows reconstitution experiments in FVIII-/- C57BI/6 mice in accordance with an embodiment of the present invention.
   Panel A displays the reconstitution with Factor VIII in the absence or presence of inhibitor Ab BO2C11; Panel B shows the dose dependent neutralisation by Ab 14C12 of BO2C11-mediated FVIII inhibition. Ab BO2C11 and Ab 14C12 were preincubated; in both Panel C and B, Ab14C12 administration was followed by BO2C11 and FVIII.

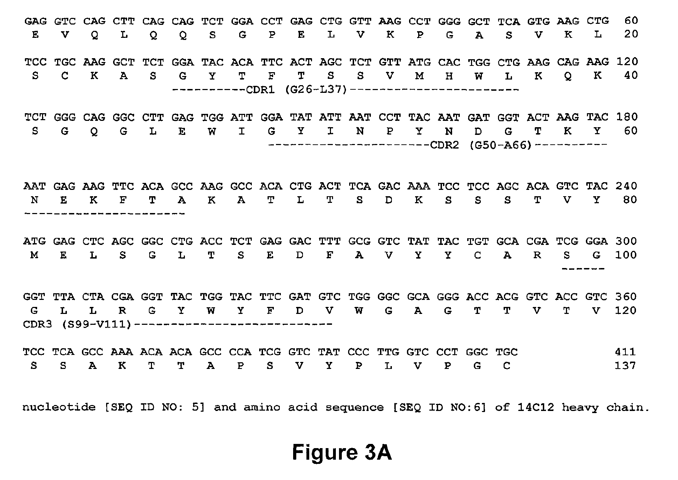

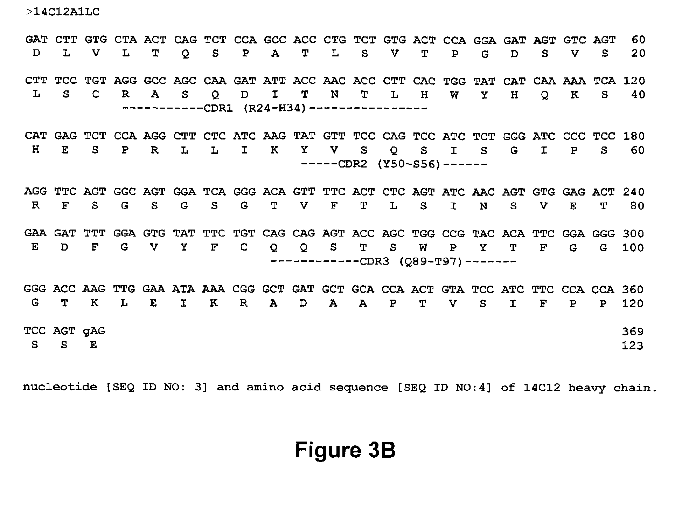

**Figure 3** shows the nucleotide and amino acid sequence of the heavy and light chain of anti-idiotypic antibody 14C12. The CDR regions are indicated under the amino acid sequence.

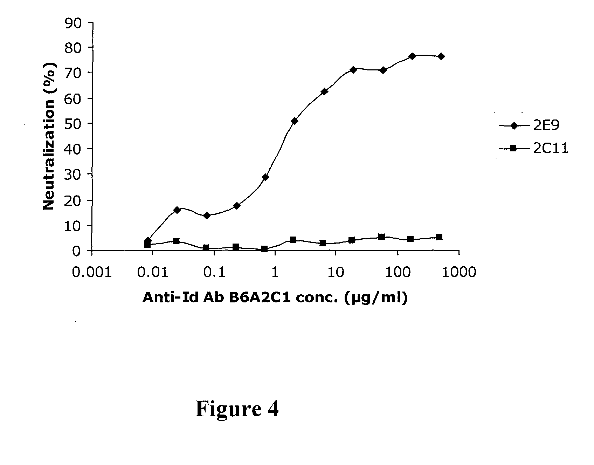

**Figure 4** shows the neutralisation of FVIII binding to human inhibitory antibodies against the C1 domain (2E9) and the C2 domain (BO2C11) by anti-Idiotypic mouse MoAb against an inhibitor to the C1 domain (B6A2C1).

### DEFINITIONS

**Factor VIII** abbreviated as **FVIII** refers to the glycosylated blood coagulation factor with a molecular weight of 330kD of 2332 amino acids which undergoes proteolytic processing.
**Light chain of FVIII** refers the proteolytic processed part of FVIII containing A3, C1 and C2 domain.
**C2 domain** refers to the fragment between residues 2173 and 2332 of FVIII.
**Phospholipid binding site** refers to a region in the C2 domain between amino acids 2302 and 2332.
**Capacity to neutralise the inhibition** refers to a property of anti-idiotypic antibodies of the present invention as determined by assaying the FVIII activity in the presence of inhibitor and anti-idiotypic Ab in an assay such as the Factor VIII chromogen test as described in Jacquemin et al. (1998) *Blood* **92,** 494-506.
**Idiotope** refers to a single antigenic determinant.
**Idiotype** refers to the collection of idiotopes within the variable region that confers on an immunoglobulin molecule an antigenic individuality and is frequently an attribute of a given antoby in an individual.
**Internal image of an epitope** are those idiotypes which conformationally mimic an antigenic epitope.
**Anti-idiotypic antibody** in the present invention refers to a second generation antibody (Ab2), which can be polyclonal but is preferably monoclonal and directed towards the variable part of pathogenic antibodies (Ab1) and which have the theoretical capacity to neutralise Ab1 activity and production. Ab1 in the present invention refers to inhibitory antibodies against the C2 domain of Factor VIII. Ab1 can be from mice, guinea pigs, horses, goats, non human primates and other mammals, but is preferably a human inhibitory antibody against human Factor VIII.
**Complementarity determining regions (CDR)** in the present invention refers to the hypervariable amino acid sequences within antibody variable regions which interact with complementary amino acids on the antigen. In one embodiment of the present invention the CDR regions are the CDR1, CDR2 and CDR3 region of the variable light and heavy chains of the Ab 14C12.
The term "**Antibody**" refers to intact molecules as well as fragments thereof, such as Fab, Fab', F(ab') 2 or , which are capable of binding to the epitope determinant of the relevant factor or domain of the factor. **F(ab')2** refers the Igg fragment obtainable after pepsin cleavage and is built up from both light chains and parts of the heavy chains disulfide linked via the hinge region. The **Fab** fragment is obtainable from the F(ab')2 by papain digestion of the hinge region and contains a one light chain and one part of the heavy chain.
"**Humanized antibod**y" as used herein, refers to antibody molecules in which amino acids have been replaced in the non-antigen binding regions in order to more closely resemble a human antibody.
A **"Reshaped human antibody" or a"Human hybrid antibody"** as used herein, refers to a human antibody in which amino acids in the antigen binding regions have been replaced with sequences in accordance with the present invention, e. g. CDR's, or other parts of variable regions which have been derived from the repertoire of human antibodies.
**Sequence comparisons.** Where in accordance with the present invention comparisons are made between amino acid sequences of two VH chains or of two VL chains or of two CDR regions, or comparisons are made between two nucleotide sequences encoding VH, VL, or CDR region, the level of sequence identity between two sequences may include having at least 80%, preferably at least 80% more preferably at least 90%, even more preferably at least 95% and most preferably at least 99% sequence identity in between two sequences.
"**modified**" denotes any protein (or polypeptide) molecule in which a single or a number of amino-acids have been either substituted by any other amino-acid residue or deleted. Such amino-acid substitution or deletion can be located anywhere in the protein molecule. It also denotes protein molecules in which amino-acid residues have been substituted and/or deleted at more than a single location. In the latter case, any combination of substitution and deletion can be considered. It also refers to polymorphisms (i.e. the regular and simultaneous occurrence in a single interbreeding population of two or more alleles of a gene, where the frequency of the rarer alleles is greater, typically greater than 1% than can be explained by recurrent mutation alone.)

### DETAILED DESCRIPTION OF THE INVENTION

The present invention will be described with reference to certain embodiments and to certain figures but the present invention is not limited thereto but only by the claims.

The present invention provides cell lines producing monoclonal antibodies which are reactive against FVIII inhibitory antibodies that are directed against the C2 domain of FVIII. The present invention also provides fragments of any of the above monoclonal antibodies such as Fab, Fab', F (ab') 2, , CDR's, single variable domains as well as modified versions thereof and combinations of these fragments and modifications. Antibodies where the respective CDR sequences are different in one arm compared to the other arm of the antibody are also within the scope of the invention.

The present invention further provides reshaped monoclonal antibodies or human hybrid monoclonal antibodies against FVIII inhibitory antibodies directed against the C2 domain of FVIII. By human hybrid monoclonal antibodies it is meant a hybrid antibody constructed from a human antibody and from variable regions in accordance with the present invention.

The present invention further provides monoclonal anti-idiotypic antibodies being produced by on purpose immunisation in animals, preferably in mouse, for instance by injecting human FVIII inhibitory antibodies (such as the inhibitory antibody BO2C11 directed against the C2 domain of FVIII) in mice and then fusing the spleen lymphocytes with a mouse myeloma cell line, followed by identifying and cloning the cell cultures producing anti-factor VIII antibodies. The monoclonal antibodies produced in animals can then humanized, for instance by associating the binding complementarity determining region (CDR) from the non-human monoclonal antibody with human framework regions -in particular the constant C region of human gene-such as disclosed by Jones et al. in *Nature* (1986) **321:** 522 or Riechmann in *Nature* (1988) **332**: 323.

An embodiment of the present invention is provided by the deposited cell line 14C12 Accession Number LMBP 5878CB producing the anti idiotypic antibody 14C12.

The present invention also provides fragments and modified versions of the protein, in particular fragments comprising complementarity determining regions ("CDR's") of the above monoclonal anti-iditioypic antibodies as well as modified versions thereof. For instance, the invention provides antigen-binding fragments Fab, Fab' and F (ab')2 generated by proteolytic digestion of the said monoclonal antibodies using methods well known in the art, such as described by Stanworth et al., Handbook of Experimental Immunology (1978), vol. 1 chapter 8 (Blackwell Scientific Publications). Such fragments, which contain the antibody binding site, have lost a number of properties of the parent antibody, such as complement activation or capacity to bind to Fc gamma receptors, however without losing their ability to inhibit inhibitory antibodies against FVIII. The present invention also includes single chain fragment variables (scFv), single variable domain fragments of the antibodies and combination of these fragments and of the above mentioned fragments.

The present invention also provides soluble or membrane anchored single-chain variable parts of the above monoclonal antibodies and a method for their obtention as follows.The DNA sequences of the variable parts of human heavy and light chains are amplified in separated reactions and cloned. A fifteen amino-acid linker sequence, for instance (Gly4 Ser) 3, is inserted between VH and VL by a two-step polymerase chain reaction (PCR), for instance according to Dieffenbach and Dveksler, "PCR Primer, a laboratory manual" (1995), Cold Spring Harbour Press, Plainview, NY, USA. The resulting fragment is then inserted into a suitable vector for expression of single chain fragment variable (scFv) as a soluble or phage-displayed polypeptide. This can be achieved by methods well known to those skilled in the art, such as described by Gilliland et al., *Tissue Antigens* (1996) **47:** 1-20. The present invention also includes a ligand comprising peptides representative of hypervariable regions of a monoclonal antibody which can be obtained by synthesis using an applied biosystem synthesiser, for instance a polypeptide synthesiser such as model 9050 available from Milligen (USA) or a model from a related technology, which alone or in combination with other or similar hypervariable regions will exert properties similar to that of the parent antibody. Fragments and peptides can be generated by recombinant DNA technology. Peptides with a length up to about 100 amino acid sequences can be made by synthetic peptide synthesis such as BOC *(tert* butyloxycarbonyl) or FMOC (9-fluorenylmethoxycarbonyl) synthesis and other techniques known in the art. Optionally the amino and carboxy groups can be chemically changed in order to increase the stability and lifetime of the peptide (e.g. formylation, acetylation). Alternatively the peptide can be covalently or non-covalently absorbed to a carrier.

The present invention further provides a pharmaceutical composition for the prevention or treatment of bleeding in a haemophilia patient having inhibitory antibodies against the C2 domain of FVIII in humans, comprising, as an active ingredient, the monoclonal anti-idiotypic antibody and fragments and modified versions such as disclosed hereinabove, in admixture with a pharmaceutically acceptable carrier. More preferably the said monoclonal antibody is a monoclonal antibody, or a fragment, modified version or a sequence with high percentage of sequence similarity thereof, obtainable from the cell line 14C12 deposited with the Belgian Co-ordinated Collections of Microorganisms Accession Number LMBP 5878CB. A sequence compared to with the said monoclonal antibody is preferably at least 80% identical, more preferably at least 90% identical and even more preferably at least 95 % identical and most preferably at least 99 % identical, and the sequence identity is preferably particularly in respect to the complementarity determining regions of the antibody. A ligand in accordance with the present invention may also include a synthetic polypeptide of equivalent potency. The pharmaceutical composition of the present invention should comprise a therapeutically effective amount of the said above ingredient, such as indicated hereinafter in respect to the method of treatment or prevention.

Suitable pharmaceutical carriers for use in the pharmaceutical compositions of the invention are described for instance in Remington's Pharmaceutical Sciences 16 ed. (1980) and their formulation is well known to those skilled in the art. They include any and all solvents, dispersion media, coatings, antibacterial and antifungal agents (for example phenol, sorbic acid, chlorobutanol), isotonic agents (such as sugars or sodium chloride) and the like. Additional ingredients may be included in order to control the duration of action of the monoclonal antibody active ingredient in the composition.

Control release compositions may thus be achieved by selecting appropriate polymer carriers such as for example polyesters, polyamino acids, polyvinyl pyrrolidone, ethylene-vinyl acetate copolymers, methylcellulose, carboxymethylcellulose, protamine sulfate and the like. The rate of drug release and duration of action may also be controlled by incorporating the monoclonal antibody active ingredient into particles,e. g. microcapsules, of a polymeric substance such as hydrogels, polylactic acid,hydroxymethylcellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition comprising the active ingredient may require protective coatings. The pharmaceutical form suitable for injectionable use include sterile aqueous solutions or dispersions and sterile powders for the extemporaneous preparation thereof. Typical carriers therefore include biocompatible aqueous buffers, ethanol, glycerol, propylene glycol, polyethylene glycol and mixtures thereof.

The present invention also provides the use as a medicament of a monoclonal anti-idiotypic antibody against FVIII inhibitory antibodies directed against the C2 domain of FVIII. More preferably the medicament used in the present invention is a means for reducing and/or preventing and/or treating bleeding in a haemophilia patient with inhibitory antibody against the C2 domain of FVIII. The said ligand may be provided to a patient by any means well known in the art, e.g. intravenously, intraarterially, parenterally or by catheterization.

The present invention therefore provides a method of treatment and/or prevention for preventing and/or treating and/or reducing the bleeding in a haemophilia patient having inhibitory antibody against the C2 domain of FVIII in a mammal, preferably a human, comprising administering to a mammal in need of such treatment or prevention or attenuation of coagulation a therapeutically effective amount of a ligand as disclosed hereinabove. Preferably the said ligand is a monoclonal antibody obtainable from cell line 14C12 or an antigen-binding fragment Fab, Fab' or F (ab') 2, or one or more peptides comprising a complementarity determining region (CDR), a soluble or membrane-anchored single-chain variable part (scFv), a single variable domain or a modified version or combination of any of these elements.

A therapeutically effective amount as used herein means from about 1 microgram to about 10 milligrams per kilogram of body weight, more preferably from about 10 micrograms to about 1 milligram per kilogram of body weight, even more preferably from about 1 to about 10 milligram per kilogram of body weight of the mammal to be treated. It will be appreciated that, in view of the long half-life time of most IgG antibodies, the ligands of the present invention which are monoclonal antibodies of the said class will enjoy a periodicity of treatment which participates in the comfort of the patient.

Haemophilia A patients with inhibitor frequently produce antibodies towards the FVIII C2 domain. The ligands of the present invention such as the antibody produced by the cell line 14C12 are therefore useful for the treatment of such patients, either in emergency situations, as for instance before a surgical procedure, or as a longer term treatment in which Ab 14C12 can induce an apoptosis of B cells carrying the corresponding anti-C2 antibodies.

The ligands of the present invention, such as the anti-idiotypic antibody AB 14C12 of cell line 14C12, have the capacity to neutralise in a functional coagulation assay by at least 50 %, preferably by at least 70 %, more preferably by at least 80 % and most preferably by at least 90%, the FVIII inhibitory properties of inhibitory antibodies against the C2 domain of FVIII.

The reader skilled in the art will understand that from the knowledge of the sequence of AB 14C12 and that of the crystal structure of BO2C11 Fab fragment in combination with the C2 domain, several variants of Ab 14C12 can be envisioned. Thus, point mutations can be introduced in the Ab 14C12 VH region to reinforce the internal image of C2, thereby reinforcing the inhibitory capacity of Ab 14C12 on C2 binding of BO2C11 and the like. Alternatively, the sequence can be altered in such a way as to increase the number of contact residues in between BO2C11 and Ab 14C12, with or without maintenance of the C2 internal image. A particular aspect of this approach is to derive a 14C12 mutant antibody which would contain contact residues recognising amino acids included in the framework regions of BO2C11. The latter belongs to the DP5 heavy chain subfamily gene segments (Jacquemin MG et al in (1998) *Blood* 92: 496-506) as other antibodies with similar C2 binding properties. Since the frequency of DP5 family members is very low in the human repertoire, neutralisation of the entire DP5 subfamily can be achieved using Ab 14C12, thereby neutralising all inhibitory antibodies of which the VH domains encoded by the DP5 VH gene segment derived from the Vh1 gene family (Jacquemin et al (1998) cited supra; Van den Brink et al in (2000) *Blood* **99,** 2828-2834.

According to the general knowledge of one skilled in the art, various different synthetic peptides can be prepared from the antibody 14C12. One or more of them VH, which alone or in combination can be used to neutralise BO2C11-like antibodies in vivo. In a preferred embodiment these peptides are derived from complementarity determining regions (CDR) of the heavy chain of the Ab 14C12 and are represented by the peptides with amino acid sequence GYTFTSSVMHWL [SEQ ID NO:5], GYINPYNDGTKYNEKFTA [SEQ ID NO:6] and SGGLLRGYWYFDV [SEQ ID NO:7]. In another preferred embodiment these peptides are derived from complementarity determining regions (CDR) of the light chain of the Ab 14C12 and are represented by the peptides with amino acid sequence RASQDITNTLH [SEQ ID NO :8], YVSQSIS [SEQ ID NO:9] and QQSTSWPYT [SEQ ID NO:10]. Thus, in one embodiment, synthetic peptides derived from Ab 14C12 complementarity regions (CDR) are used to infuse patients with inhibitory antibodies against the C2 domain of FVIII prior to surgery. The peptides combine with inhibitory antibodies and neutralise their inhibitory capacities. In a yet alternative embodiment, a recombinant polypeptide is made by combining the sequence of different CDRs, with appropriate linker polypeptide sequences to maintain a suitable 3-D conformation. The polypeptide is administered in patients prior to an emergency procedure such as surgery.

An additional application of Ab 14C12 is its use to induce apoptosis of B cells carrying anti-C2 antibodies. Such B cells, as memory cells or as plasmocytes, express a surface antibody equivalent to its secreted form. Crosslinking of idiotypes at the B cell surface transduces a signal leading to inhibition of proliferation or apoptosis of the cell.

The present invention is further described by the following examples which are provided for illustrative purposes only.

### EXAMPLE 1: Generation of a monoclonal Ab against FVIII inhibitor BO2C11.

The Factor VIII specific human Ig4kappa BO2C11 antibody was used as for the generation of anti-idiotypic antibodies in mice. The properties of BO2C11 are described in Jacquemin et al (1998) *Blood* **92,** 496-506. The nucleotide and amino acid sequences of the variable light and heavy chain of BO2C11 are disclosed in PCT patent application WO01/04269.

Balb/c mice were immunised in the footpad with BO2C11 emulsified in first complete and then incomplete Freund's adjuvant. After 4 such injections, mouse serums were tested in an ELISA system for the presence of antibodies recognising BO2C11, but not other anti-FVIII antibodies or unrelated antibodies of the same isotype. Splenocytes from mice producing specific anti-BO2C11 antibodies were fused with a myeloma cell lines to produce B cell clones (Kohler G et al. in (1978). *Eur J* Immunol **8 :** 82-88.). These were expanded in culture and tested for BO2C11 specificity. One clone, 14C12, effectively bound to polystyrene plates coated with BO2C11.

The cell line 14C12 was deposited on July 30, 2002 at the Belgian Coordinated Collections of Microorganisms (BCCM), LMBP (plasmid collection, Laboratorium voor Moleculaire Biologie, Universiteit, K.L. Ledeganckstraat 35, 9000 Gent, Belgium) with Accession Number LMBP 5878CB..

### EXAMPLE 2: In vitro properties of the anti-idiotypic antibody obtained from cell line 14C12.

The properties of the anti-idiotypic antibody (Ab 14C12) obtained from 14C12 cell line were examined in in vitro assay systems. Ab 14C12 was shown to bind to BO2C11 and to inhibit in a dose-dependent manner the binding of BO2C11 to its target antigen, the FVIII C2 domain. Moreover, the capacity of Ab 14C12 to neutralise the FVIII inhibitory properties of BO2C11 in a functional coagulation assay was also assessed. The chromogenic (Faktor VIII chromogen test, Dade Behring, Marburg, Germany) is based on the conversion of a colourless substrate, which is specifically cleaved by thrombin. The test system contains every reagent required to produce thrombin, except FVIII. The intensity of colour development over time is therefore proportional to the amount of FVIII added to the system. Addition of BO2C11 (0.1 µg/ml) completely inhibits the FVIII-dependent conversion (0,3 IU/ml) of the substrate.BO2C11 was added to the chromogenic assay at a final concentration of 0,1 µg/ml, which was determined as the minimum concentration of BO2C11 able to inhibit 100% of FVIII (0,3 IU/ml) in the test system. Different concentrations of Ab 14C12 were preincubated with this fixed amount of BO2C11 before addition of FVIII. The mixture was then applied to the chromogenic assay.

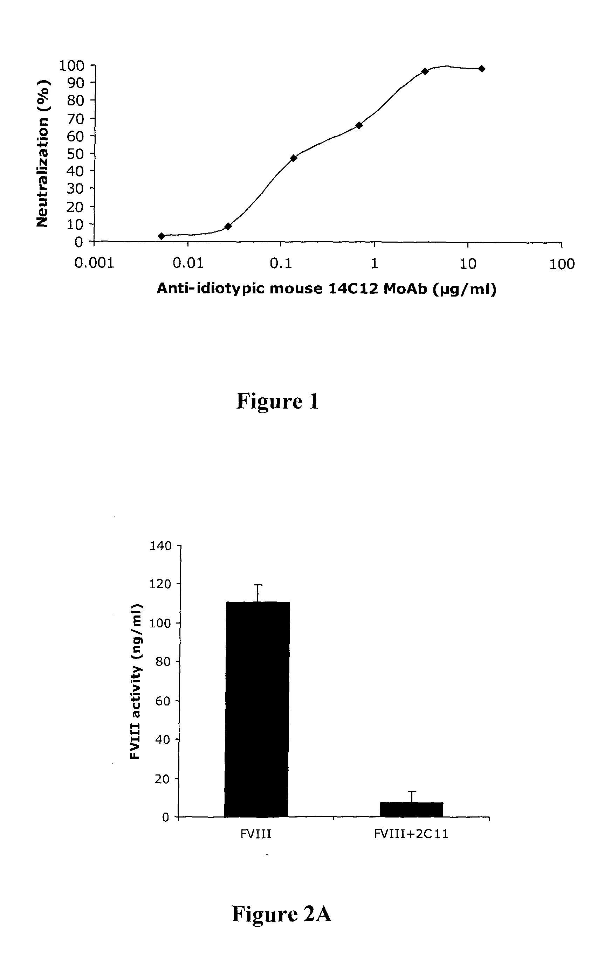

Figure 1 shows that addition of Ab 14C12 to the test system neutralises in a dose-dependent manner the inhibitory capacity of BO2C11. Moreover, 50% neutralisation is obtained at a 1/1 molar ratio with BO2C11.

The capacity of Ab 14C12 to neutralise polyclonal anti-FVIII antibodies was tested in a chromogenic assay as described above. To this end, polyclonal antibodies of the patient from whom BO2C11 was derived were added to the system as a substitute for BO2C11. Under such conditions, up to 60% neutralisation was obtained upon addition of serial dilutions of Ab 14C12. It is known that such a polyclonal antibody preparation contains a significant amount of antibodies directed towards the heavy chain of FVIII, i.e. towards epitopes fully distinct from the C2 domain. It was therefore concluded that the 60% neutralisation of inhibition observed with polyclonal antibodies is an underestimation of Ab 14C12 capacity to neutralise anti-C2 inhibitor.

In addition, Ab 14C12 significantly neutralised the inhibitory properties of polyclonal antibodies from unrelated patients. Thus, polyclonal antibodies were added at different dilutions to an immunoprecipitation assay in which radiolabelled FVIII domains are produced by combined transcription/translation using rabbit reticulocytes (Promega kit, TNT couplet reticulocyte lynate, Madison, USA). The trace amount of radiolabelled domains produced is mixed with antibody samples and protein A Sepharose. Sepharose beads are added at a concentration at which essentially all antibodies are captured. After centrifugation and washing, the radioactivity is counted on the samples, which is proportional to the presence of antibodies with specificity for the FVIII fragment tested. Thus, polyclonal antibodies of 6 unrelated patients with haemophilia A and inhibitor towards the C2 domain were added at defined concentration in an assay in which the C2 domain was translated. Ab 14C12 was then added, at increased concentration, to all samples. Three out of the 6 samples yielded significant neutralisation (circa 50%) of the inhibitory properties of polyclonal antibodies, showing that Ab 14C12 recognises an epitope commonly expressed on human anti-C2 antibodies.

### Example 3: in vivo properties of Ab 14C12.

The capacity of Ab 14C12 to neutralise the FVIII inhibitory property of BO2C11 in vivo was examined in FVIII-/- C57BI/6 mice reconstituted with human recombinant FVIII (Singh I et al. in (2002) *Blood* **99**: 3235-3240).

Such mice are considered to be a suitable animal model for haemophilia A, insofar as no FVIII activity or antigen can be detected, with an otherwise normal phenotype (Reipert BM et al. in *Thromb Haemost* 2000; **84**: 826-32). Administration of 1 IU human FVIII in the tail vein of FVIII-/- C57BI/6 mice resulted in a plasma concentration of 110 ng/ml after 10 min, namely ± 60% of the normal FVIII concentration in human plasma (1 IU or 192 ng/ml). When such mice are first injected with 0.5 µg of BO2C11, FVIII procoagulant activity is inhibited by 98%. The model was then used to determine whether Ab 14C12 had the capacity to neutralise the BO2C11-dependent inhibition of FVIII. Thus, different concentrations of Ab 14C12 were either mixed with a fixed amount of BO2C11 (0.5 µg) before injection of the complex in FVIII-/- mice, or directly infused in mice, followed by 0.5 µg of BO2C11 and 1 IU FVIII.

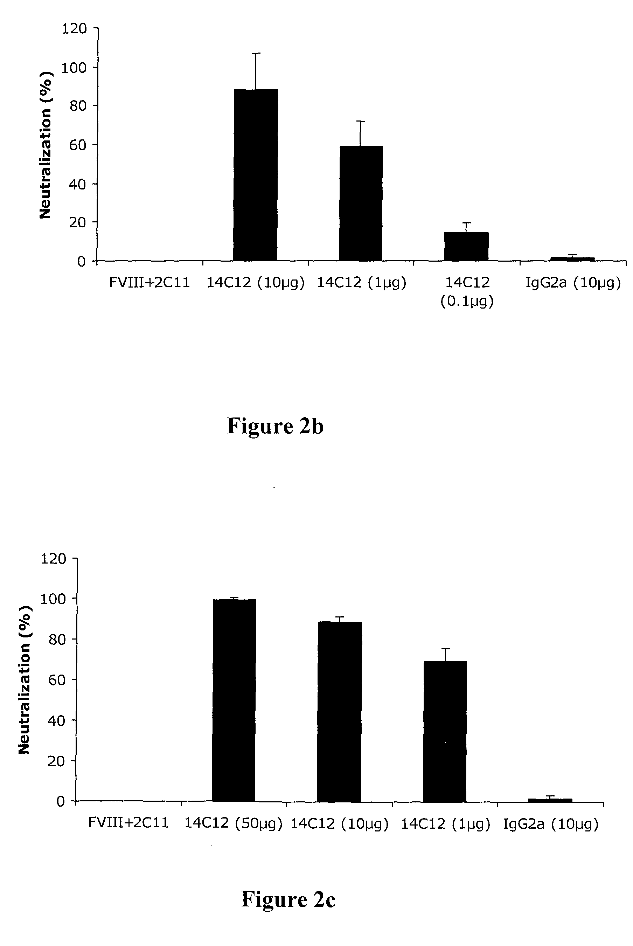

Pre-administration of 0.5 µg BO2C11 inhibited 98% of the FVIII procoagulant activity (Fig 2 A). The fixed amount of BO2C11 was preincubated with various concentrations of Ab 14C12 (0.1 to 10 µg) and the mixture infused in FVIII-/- mice. A dose-dependent neutralization of BO2C11-mediated FVIII inhibition was observed (Fig 2B). The experiment was repeated but with sequential administration of Ab 14C12 followed by BO2C11 and FVIII, yielding an identical dose-dependent 14C12 mediated neutralisation (Fig 2C).

It can be seen from Figures 2A, 2B and 2C that Ab 14C12 neutralises in a dose-dependent manner the inhibition properties of BO2C11, confirming thereby the potential of Ab 14C12 as a therapy for haemophilia A patients with inhibitor towards the C2 domain of FVIII. Interestingly, as shown in in vitro assays, 50% neutralisation was obtained at an equimolar ratio between BO2C11 and Ab 14C12. An Ab 14C12 molar excess of 3.5 was sufficient to fully neutralise BO2C11 and restoring a normal FVIII procoagulant activity.

### Example 4: Binding characteristic of Ab 14C12 to BO2C11.

A thorough biochemical evaluation of Ab 14C12 was carried out. Ab 14C12 binds with high affinity to BO2C11, with kₒₙ and k_{off} values of 10⁵ m⁻¹S⁻¹, 10⁻⁵ S⁻¹ respectively, as measured using a surface plasmon resonance system. The VH and VL domains of Ab 14C12 were sequenced by conventional methods (figure 3). Thus Ab 14C12 belongs to the IJHV1 heavy chain antibody family, with a kappa light chain. The capacity of Ab 14C12 to inhibit the binding of BO2C11 to the FVIII C2 domain prompted the inventors to compare the sequence of the VH and VL domains with that of C2. The CDR1 and CDR2 of VH contain 6 and 7 amino acid residues that are identical or homologous to the C2 domain, respectively. CDR2 is almost identical to the V2223-T2237 C2 region, which contains a crucial phospholipid-binding site and a number of contact residues for BO2C11. In addition, based on the sequence of BO2C11 variable parts (Jacquemin MG et al. in (1998) *Blood* **92:** 496-506) and its crystal structure in association with C2 (Spiegel P.C. Jr. et al. in (2001) *Blood* **98:** 13-19), putative contact residues between BO2C11 and Ab 14C12 were identified, some of which are located within the framework of BO2C11 variable parts. Taken together, these data show that the variable part of the heavy chain of Ab 14C12 contains both an internal image of C2 made of 13 identical or homologous amino acid residues, and a number of contact residues for the variable part of BO2C11.

Ab 14C12 therefore represents the first example of an anti-idiotypic antibody directed towards the C2 domain of FVIII, administration of which restores FVIII activity in vivo in a murine model of haemophilia A. As such, it discloses an effective therapy for haemophilia A patients with FVIII inhibitors.

To determine whether B6A2C1 can interfere with the binding of BO2C11 to FVIII, serial dilutions of this antibody were made and tested for their capacity to inhibit the binding of BO2C11 to FVIII-coated polystyrene plates. Figure 4 shows that no inhibition occurred, indicating that B6A2C1 expressed no specificity for BO2C11. Antibody B6A2C1 is therefore clearly distinct from the present invention.

## Claims

1. A monoclonal anti-idiotypic antibody against a human Factor VIII inhibitory antibody, the said inhibitory antibody being directed towards the C2 domain of Factor VIII.

2. A monoclonal anti-idiotypic antibody according to claim 1, further **characterised in** having the capacity to neutralise by at least 50% the inhibition of FVIII procoagulant activity mediated by inhibitory antibodies against the C2 domain of FVIII.

3. A monoclonal anti-idiotypic antibody according to claim 1 or 2 wherein the said Factor VIII inhibitory antibody has a variable heavy chain of which the VH domains are encoded by the DP5 VH gene segment derived from the Vh1 gene family.

4. A monoclonal anti-idiotypic antibody according to any of claims 1 to 3 wherein the Factor VIII inhibitory antibody is BO2C11.

5. An monoclonal anti-idiotypic antibody according to any of claims 1 to 4 wherein the variable heavy chain of the said anti-idiotypic antibody is encoded by the nucleotide sequence depicted in SEQ ID NO 1 or a nucleotide sequence having at least 80% sequence identity to SEQ ID NO 1 and/or wherein the variable light chain of the anti-idiotypic antibody is encoded by the nucleotide sequence depicted in SEQ ID NO 3 or a nucleotide sequence having at least 80% sequence identity with SEQ ID NO 3.

6. A monoclonal anti-idiotypic antibody according to any of claims 1 to 5 wherein a complementary determining region of the variable heavy and light chains of said antibody has at least 80 % sequence identity to one of amino acid sequences depicted in SEQ ID NO:5, SEQ ID NO:6, SEQ ID NO:7, SEQ ID NO:8 SEQ ID NO:9 and SEQ ID NO:10.

7. A F(Ab')2 fragment, a Fab' fragment , a Fab fragment of a monoclonal anti-idiotypic antibody in accordance with any of claims 1 to 6 or a modified version of said fragment.

8. An isolated and purified peptide having an amino acid sequence selected from SEQ ID NO:5, SEQ ID NO:6, SEQ ID NO:7, SEQ ID NO:8, SEQ ID NO:9 and SEQ ID NO:10 being at least 80 % identical in amino acid sequence to a peptide with an amino acid sequence selected from SEQ ID NO:5, SEQ ID NO:6, SEQ ID NO:7, SEQ ID NO:8, SEQ ID NO:9 and SEQ ID NO:10.

9. A monoclonal cell line expressing a monoclonal anti-idiotypic antibody in accordance with any of claims 1 to 6.

10. A monoclonal cell line in accordance with claim 9 being the cell line 14C12 deposited at BCCM with Accession Number LMBP 5878CB.

11. A pharmaceutical composition comprising a monoclonal anti-idiotypic antibody according to any of claims 1 to 6, or a fragment according to claim 7 or 8, or an isolated and purified peptide according to claim 8, in admixture with at least one pharmaceutically acceptable carrier.

12. Use of a monoclonal anti-idiotypic antibody according to any of claims 1 to 6, or a fragment according to claim 7 or 8, or an isolated and purified peptide according to claim 8 as a medicine.
